# EUROPEAN PATENT SPECIFICATION

(11) **EP 2 956 554 B1**
(45) Date of publication and mention of the grant of the patent: **09.10.2019**
(21) Application number: 14751442.6
(22) Date of filing: 14.02.2014
(51) Int. Cl.: C12Q 1/6886, C07K 14/71, G01N 33/574, C07K 16/28, C07K 14/485, C12N 15/113

(54) **A NOVEL EGFR VARIANT**
NEUARTIGE EGFR-VARIANTE
NOUVEAU VARIANT EGFR

(30) Priority: 15.02.2013 US 201361765537 P
(43) Date of publication of application: 23.12.2015
(73) Proprietor: Exosome Diagnostics Inc., New York, NY 10032 (US)
(72) Inventor: SKOG, Johan, Karl, Olov, Charlestown, MA 02129 (US); BERGHOFF, Emily, Cambridge, MA 02139 (US); LOGUIDICE, Lori, Cambridge, MA 02139 (US)
(74) Representative: Cooley (UK) LLP
(86) International application number: PCT/US2014/016536
(87) International publication number: WO 2014/127266

(56) References cited:
- US-A1- 2006 134 663
- US-A1- 2010 120 628
- A. IDBAIH ET AL: "Epidermal growth factor receptor extracellular domain mutations in primary glioblastoma", NEUROPATHOLOGY AND APPLIED NEUROBIOLOGY., vol. 35, no. 2, 1 April 2009 (2009-04-01), pages 208-213, XP055297254, GB ISSN: 0305-1846, DOI: 10.1111/j.1365-2990.2008.00977.x
- JULIO C. RICARTE-FILHO ET AL: "Absence of common activating mutations of the epidermal growth factor receptor gene in thyroid cancers from American and Japanese patients", INTERNATIONAL JOURNAL OF CANCER, vol. 130, no. 9, 24 August 2011 (2011-08-24), pages 2215-2217, XP055297257, US ISSN: 0020-7136, DOI: 10.1002/ijc.26267
- HUANG ET AL.: 'Oncogenic EGFR signaling networks in glioma.' SCI SIGNAL vol. 2, no. 87, 08 September 2009, pages 1 - 13, XP055271166
- WANG ET AL.: 'Identification of an exon 4-deletion variant of epidermal growth factor receptor with increased metastasis-promoting capacity.' NEOPLASIA vol. 13, no. 5, May 2011, pages 461 - 471, XP055271168
- YAMAZAKI ET AL.: 'A deletion mutation within the ligand binding domain is responsible for activation of epidermal growth factor receptor gene in human brain tumors.' JPN J CANCER RES vol. 81, no. 8, August 1990, pages 773 - 779, XP055271205
- MOSCATELLO D K ET AL: "FREQUENT EXPRESSION OF A MUTANT EPIDERMAL GROWTH FACTOR RECEPTOR INMULTIPLE HUMAN TUMORS", CANCER RESEARCH, AMERICAN ASSOCIATION FOR CANCER RESEARCH, US, vol. 55, 1 December 1995 (1995-12-01), pages 5536-5539, XP002943271, ISSN: 0008-5472
- S K Batra ET AL: "Epidermal growth factor ligand-independent, unregulated, cell-transforming potential of a naturally occurring human mutant EGFRvIII gene", Cell growth & differentiation : the molecular biology journal of the American Association for Cancer Research, 1 October 1995 (1995-10-01), pages 1251-1259, XP055367769, UNITED STATES Retrieved from the Internet: URL:http://cgd.aacrjournals.org/cgi/reprin t/6/10/1251 [retrieved on 2017-04-26]

## Description

### FIELD OF INVENTION

The present disclosure relates generally to a novel EGFR variant, to methods for detecting the novel EGFR variant, and to methods for diagnosing and treating cancer.

### BACKGROUND

Growth factors and their receptors play important roles in modulating cell division, proliferation and differentiation. As a result, many growth factor receptors are mutated or aberrantly expressed in cancers, causing deregulation of the growth factor pathways, and resulting in the uncontrolled proliferation that is characteristic of cancer.

Epidermal growth factor receptor (EGFR) is among those growth factor receptors that are frequently mutated, aberrantly expressed, or misregulated in cancers. In certain cancers, such as glioblastoma multiforme (GBM), up to 50% of patients exhibit tumor-specific EGFR variants, such as EGFRvIIl. Mutation and amplification of EGFR have also recently been implicated in the oncogenesis and progression ofhuman solid tumors and epithelial tumors and correlated with shorter survival time/rate. Therefore, EGFR has emerged as valuable a biomarker for cancer diagnosis and prognosis. Various EGFR mutations are known (Huang et al., Sci Signal (2009) 2: 1-13; Wang et al., Neoplasia (2011) 13, 461-471; Idbaih et al., Neuropathology and applied Neurobiology (2009) 35, 208-213; Ricarte-Filho et al., International Journal of Cancer (2011) 130, 2215-2217; Yamazaki et al., Jpn J Cancer Res (1990) 81, 773-779; Moscatello et al., Cancer Research, American Association for Cancer Research (1995) 55, 5536-5539; Batra et al., Cell Growth & Differentiation : the Molecular Biology Journal of the American Association for Cancer Research (1995) 1251-1259; US2006/134663).259; US 2006/134663).

Importantly, EGFR has also been targeted for anti-cancer therapeutics, with the development of therapies directed towards eliminating the EGFR signal transduction. Two classes of therapies curently exist, antibodies that prevent ligand-mediated EGFR activation and small molecules that inhibit EGFR kinase activity. However, clinical data reveals that not all patients respond to such therapies, which may be a result of tumor-specific expression of different EGFR variants. The EGFRvIII variant lacks a portion of the N-terminal ligand-binding domain. As a result, GBM patients with tumors that express EGFRvIII have been found to be nonresponsive to therapies that specifically target the N-terminal ligand-binding domain. Detection of particular EGFR variants has shown utility for predicting responsiveness of a patient to particular EGFR-targeted therapies.

Accordingly, identification of different cancer-associated EGFR variants is useful to provide improved diagnosis, prognosis, stratification and/or therapy guidance of a cancer.

### SUMMARY OF THE INVENTION

This disclosure is based on the discovery of a novel EGFR variant, identified in a glioblastoma patient. The present description addresses the need for cancer biomarkers for more accurate diagnosis of cancer and the predisposition for cancer, as well as biomarkers to guide therapeutic regiments for treatment of cancer. In general, there is featured a novel EGFR variant, referred to herein as EGFRvVI. The disclosure also relates to detection of the EGFR variant for the diagnosis, prognosis, and/or therapy guidance of a cancer.
In a first aspect of the invention, there is provided an isolated epithelial growth factor receptor (EGFR) protein comprising an in-frame deletion that starts in exon 3 and ends in exon 7 of wild-type EGFR, wherein amino acids 90-221 of wild-type EGFR have been deleted and wherein said EGFR protein comprises the amino acid sequence of SEQ ID NO: 5 or an amino acid sequence having greater than 90% identity to the amino acid sequence of SEQ ID NO: 5.
In a second aspect of the invention, there is provided an antibody that specifically binds to an EGFR protein encoded by a polynucleotide comprising the nucleotide sequence of SEQ ID NO: 3 or SEQ ID NO: 4, or that binds an EGFR protein comprising the amino acid sequence of SEQ ID NO: 5 or an amino acid sequence having greater than 90% identity to the amino acid sequence of SEQ ID NO: 5, but does not bind to wild-type EGFR and other EGFR variants.
In a third aspect of the invention, there is provided an agent that specifically binds to an EGFR protein encoded by a polynucleotide comprising the nucleotide sequence of SEQ ID NO: 3 or SEQ ID NO: 4, or that binds an EGFR protein comprising the amino acid sequence of SEQ ID NO: 5 or an amino acid sequence having greater than 90% identity to the amino acid sequence of SEQ ID NO: 5, but does not bind the wild-type EGFR and other EGFR variants, for use in treating cancer, wherein said agent is an antibody.
In a fourth aspect of the invention, there is provided a specific inhibitor of an EGFR protein encoded by a polynucleotide comprising the nucleotide sequence of SEQ ID NO: 3 or SEQ ID NO: 4, or that inhibits an EGFR protein comprising the amino acid sequence of SEQ ID NO: 5 or an amino acid sequence having greater than 90% identity to the amino acid sequence of SEQ ID NO: 5, but does not inhibit wild-type EGFR and other EGFR variants for use in treating a cancer wherein said inhibitor is a RNAi agent or antibody.

In any of the methods described herein, the EGFR inhibitor may be a small molecule, an antibody, a nucleotide, an RNA interfering agent. The EGFR inhibitor inhibits or decreases EGFR activity or mRNA or protein expression. Examples of EGFR inhibitors include, but are not limited to, gefitinib (IRESSA™), erlotinib (TARCEVA®), cetuximab (ERBITUX®), lapatinib (TYKERB®), panitumumab (VECTIBIX®), vandetanib (CAPRELSA®). Other examples of EGFR inhibitors include cancer vaccines that target EGFR
deletions, such as Rindopepimut (CDX-110, Celldex Therapeutics). Other inhibitors that can be used with the present disclosure are PARP1 inhibitors.

In the methods described herein for determining the presence of the EGFR variant, the RNA, DNA or cDNA sequence of the EGFR variant is detected, for example by nucleic acid amplification or quantitative real-time PCR. The amplification product or levels of the EGFR variant can be compared to a reference sample, wherein the reference sample is from a subject does not express EGFRvVI, or expresses wild-type EGFR. The reference sample may be from a subject that does not have cancer. The reference sample may be from the same subject at an earlier timepoint, e.g., before starting a therapy regimen, or before diagnosis of cancer. Changes in the levels or presence of EGFRvVI for a particular subject may aid selection of a therapy or assessment of the efficacy of a therapy.

The present disclosure also provides for the use of an agent that specifically binds to or recognizes the EGFRvVI variant in an *ex vivo* method for targeting a cancer cell that expresses the EGFR protein of the invention. Additionally, there is provided an agent that specifically binds the EGFR protein for use in treating cancer, wherein the agent targets a cancer cell that expresses the EGFR protein if the invention. Preferably, the agent binds to or recognizes the EGFRvVI variant but does not bind to or recognize wild- type EGFR. Thus, the agent specifically recognizes cancer cells. The agent may be covalently linked to a therapeutic agent. For example, the therapeutic agent can be a chemotherapeutic agent, a toxin, a radioisotope, or a nanoparticle.

The present disclosure further provides an inhibitor of the EGFRvVI variant for use in treating a cancer. The EGFRvVI inhibitor is, for example, a small molecule, an antibody, or an RNA interfering agent. Preferably, the EGFRvVI inhibitor inhibits or decreases EGFR activity or mRNA or protein expression.

Various aspects and embodiments of the invention will now be described in detail. Further, unless otherwise required by context, singular terms shall include pluralities and plural terms shall include the singular.

### BRIEF DESCRIPTION OF THE DRAWINGS

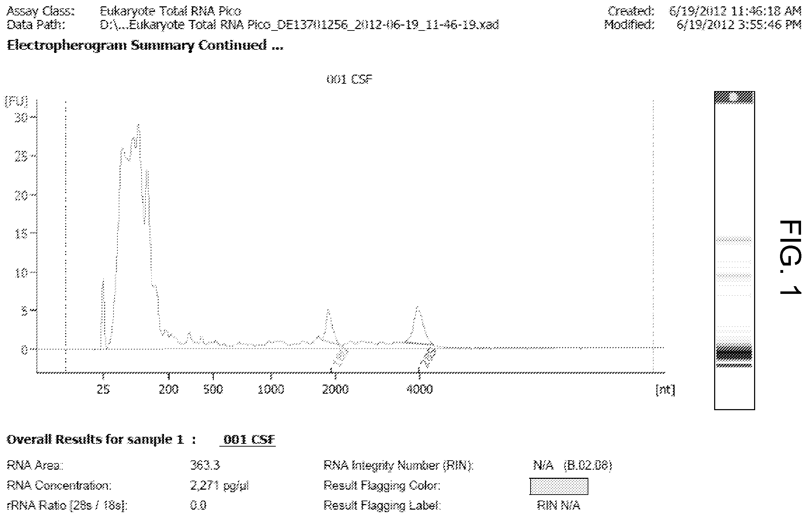

**Figure 1** shows an electropherogram of an RNA sample extracted from 4 ml of cerebrospinal fluid (CSF) sample from a single patient (patient 001). 18S and 28S rRNA peaks are labeled and used to generate the RNA integrity number (RIN).

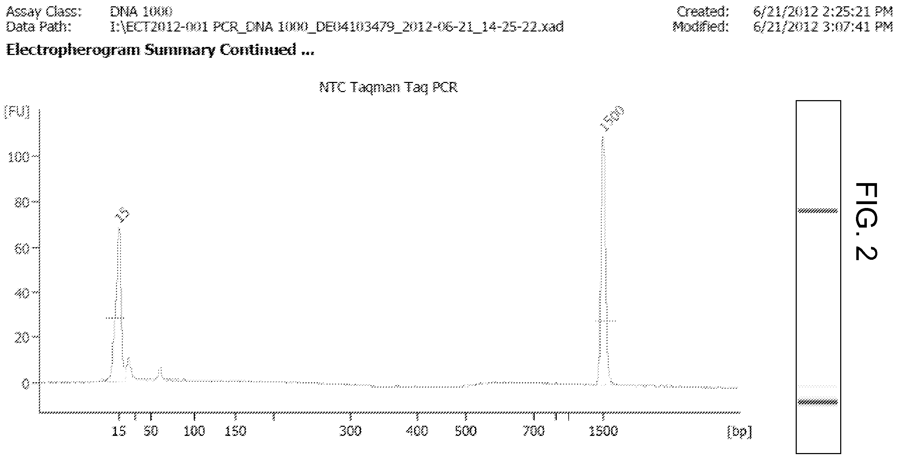

**Figure 2** shows an electropherogram of a typical control sample in the EGFR assay, where no template was used in the QPCR assay. No product is detected.
**Figure 3** shows an electropherogram of the RNA extracted from the CSF sample from patient 001 in the EGFR QPCR assay. An amplified product is detected at 397 bp.
**Figure 4** shows a schematic of the deletion at exons 3-7 of the sequenced EGFR nucleotide sequence (partial sequence shown). (A) The gray box designates the homologous region that constitutes the breakpoint in exons 3 and 7 which causes the deletion. (B) This schematic represents select exons in EGFR: exon 1 (italicized); exon 2 (bold); parts of exon 3 (underlined); parts of exon 7 (bold underlined); and exon 8 (bold italicized).
**Figure 5** shows a schematic of the EGFR variants EGFRvIII and EGFRvVI in the context of the full-length EGFR amino acid sequence. The grey box designates the region that is deleted in EGFRvVI, as detected and identified in patient 001. The underlined sequence designates the region that is deleted in EGFRvIII.

### DETAILED DESCRIPTION OF THE INVENTION

This disclosure is based on the discovery of a novel variant of the epithelial growth factor receptor (EGFR). Mutations and overexpression of EGFR are known to be associated with a number of cancers, including lung cancer, some epithelial cancers, and glioblastoma multiforme. EGFR mutations, such as the EGFRvIII variant, commonly found in cancers cause constitutive activation of EGFR, resulting in downstream signaling that can cause uncontrolled cell division, a hallmark of cancer. As a result, presence of a mutant form of EGFR can indicate of the presence of cancer or a higher predisposition for cancer. In addition, recent therapeutics have been designed to target EGFR specifically to inhibit EGFR activity. Cancer patients with certain EGFR mutations are often unresponsive to those therapeutics that act through binding or inhibition of the domain or region that is mutated. Thus, EGFR mutant variants are useful for diagnosing whether a subject has cancer or a higher predisposition for cancer, as well as for determining the responsiveness of a cancer patient to a particular therapeutic regimen or for determining the efficacy of a therapeutic regimen.

### EGFR and Cancer

The epidermal growth factor receptor (EGFR) is one of four receptors in the ErbB (also known as HER and Human Epidermal growth factor Receptor) signaling pathway that are present on the cell surface. EGFR is also known as ErbB-land HER1. The other members of the HER family include ErbB-2 (HER2/c-neu), ErbB-3 (HER3), and ErbB-4 (HER4).

EGFR is encoded by the *c-erbB1* proto-oncogene and has a molecular mass of 170 kDA. A wild-type (WT) EGFR nucleic acid can be encoded by the following human mRNA sequence (GenBank Accession No. BC094761.1) (SEQ ID NO: 1):

GTCCGGGCAGCCCCCGGCGCAGCGCGGCCGCAGCAGCCTCCTCCCCCCGCACGGTGTGAGCGCCCGCCGC
GGCCGAGGCGGCCGGAGTCCCGAGCTAGCCCCGGCGGCCGCCGCCGCCCAGACCGGACGACAGGCCACCT
CGTCGGCGTCCGCCCGAGTCCCCGCCTCGCCGCCAACGCCACAACCACCGCGCACGGCCCCCTGACTCCG
TCCAGTATTGATCGGGAGAGCCGGAGCGAGCTCTTCGGGGAGCAGCGATGCGACCCTCCGGGACGGCCGG
GGCAGCGCTCCTGGCGCTGCTGGCTGCGCTCTGCCCGGCGAGTCGGGCTCTGGAGGAAAAGAAAGTTTGC
CAAGGCACGAGTAACAAGCTCACGCAGTTGGGCACTTTTGAAGATCATTTTCTCAGCCTCCAGAGGATGT
TCAATAACTGTGAGGTGGTCCTTGGGAATTTGGAAATTACCTATGTGCAGAGGAATTATGATCTTTCCTT
CTTAAAGACCATCCAGGAGGTGGCTGGTTATGTCCTCATTGCCCTCAACACAGTGGAGCGAATTCCTTTG
GAAAACCTGCAGATCATCAGAGGAAATATGTACTACGAAAATTCCTATGCCTTAGCAGTCTTATCTAACT
ATGATGCAAATAAAACCGGACTGAAGGAGCTGCCCATGAGAAATTTACAGGGACAAAAGTGTGATCCAAG
CTGTCCCAATGGGAGCTGCTGGGGTGCAGGAGAGGAGAACTGCCAGAAACTGACCAAAATCATCTGTGCC
CAGCAGTGCTCCGGGCGCTGCCGTGGCAAGTCCCCCAGTGACTGCTGCCACAACCAGTGTGCTGCAGGCT
GCACAGGCCCCCGGGAGAGCGACTGCCTGGTCTGCCGCAAATTCCGAGACGAAGCCACGTGCAAGGACAC
CTGCCCCCCACTCATGCTCTACAACCCCACCACGTACCAGATGGATGTGAACCCCGAGGGCAAATACAGC
TTTGGTGCCACCTGCGTGAAGAAGTGTCCCCGTAATTATGTGGTGACAGATCACGGCTCGTGCGTCCGAG
CCTGTGGGGCCGACAGCTATGAGATGGAGGAAGACGGCGTCCGCAAGTGTAAGAAGTGCGAAGGGCCTTG
CCGCAAAGTGTGTAACGGAATAGGTATTGGTGAATTTAAAGACTCACTCTCCATAAATGCTACGAATATT
AAACACTTCAAAAACTGCACCTCCATCAGTGGCGATCTCCACATCCTGCCGGTGGCATTTAGGGGTGACT
CCTTCACACATACTCCTCCTCTGGATCCACAGGAACTGGATATTCTGAAAACCGTAAAGGAAATCACAGG
GTTTTTGCTGATTCAGGCTTGGCCTGAAAACAGGACGGACCTCCATGCCTTTGAGAACCTAGAAATCATA
CGCGGCAGGACCAAGCAACATGGTCAGTTTTCTCTTGCAGTCGTCAGCCTGAACATAACATCCTTGGGAT
TACGCTCCCTCAAGGAGATAAGTGATGGAGATGTGATAATTTCAGGAAACAAAAATTTGTGCTATGCAAA
TACAATAAACTGGAAAAAACTGTTTGGGACCTCCGGTCAGAAAACCAAAATTATAAGCAACAGAGGTGAA
AACAGCTGCAAGGCCACAGGCCAGGTCTGCCATGCCTTGTGCTCCCCCGAGGGCTGCTGGGGCCCGGAGC
CCAGGGACTGCGTCTCTTGCCGGAATGTCAGCCGAGGCAGGGAATGCGTGGACAAGTGCAACCTTCTGGA
GGGTGAGCCAAGGGAGTTTGTGGAGAACTCTGAGTGCATACAGTGCCACCCAGAGTGCCTGCCTCAGGCC
ATGAACATCACCTGCACAGGACGGGGACCAGACAACTGTATCCAGTGTGCCCACTACATTGACGGCCCCC
ACTGCGTCAAGACCTGCCCGGCAGGAGTCATGGGAGAAAACAACACCCTGGTCTGGAAGTACGCAGACGC
CGGCCATGTGTGCCACCTGTGCCATCCAAACTGCACCTACGGATGCACTGGGCCAGGTCTTGAAGGCTGT
CCAACGAATGGGCCTAAGATCCCGTCCATCGCCACTGGGATGGTGGGGGCCCTCCTCTTGCTGCTGGTGG
TGGCCCTGGGGATCGGCCTCTTCATGCGAAGGCGCCACATCGTTCGGAAGCGCACGCTGCGGAGGCTGCT
GCAGGAGAGGGAGCTTGTGGAGCCTCTTACACCCAGTGGAGAAGCTCCCAACCAAGCTCTCTTGAGGATC
TTGAAGGAAACTGAATTCAAAAAGATCAAAGTGCTGGGCTCCGGTGCGTTCGGCACGGTGTATAAGGGAC
TCTGGATCCCAGAAGGTGAGAAAGTTAAAATTCCCGTCGCTATCAAGGAATTAAGAGAAGCAACATCTCC
GAAAGCCAACAAGGAAATCCTCGATGAAGCCTACGTGATGGCCAGCGTGGACAACCCCCACGTGTGCCGC
CTGCTGGGCATCTGCCTCACCTCCACCGTGCAGCTCATCACGCAGCTCATGCCCTTCGGCTGCCTCCTGG
ACTATGTCCGGGAACACAAAGACAATATTGGCTCCCAGTACCTGCTCAACTGGTGTGTGCAGATCGCAAA
GGGCATGAACTACTTGGAGGACCGTCGCTTGGTGCACCGCGACCTGGCAGCCAGGAACGTACTGGTGAAA
ACACCGCAGCATGTCAAGATCACAGATTTTGGGCTGGCCAAACTGCTGGGTGCGGAAGAGAAAGAATACC

ATGCAGAAGGAGGCAAAGTGCCTATCAAGTGGATGGCATTGGAATCAATTTACACAGAATCTATACCCA
CCAGAGTGATGTCTGGAGCTACGGGGTGACCGTTTGGGAGTTGATGACCTTTGGATCCAAGCCATATGAC
GGAATCCCTGCCAGCGAGATCTCCTCCATCCTGGAGAAAGGAGAACGCCTCCCTCAGCCACCCATATGTA
CCATCGATGTCTACATGATCATGGTCAAGTGCTGGATGATAGACGCAGATAGTCGCCCAAAGTTCCGTGA
GTTGATCATCGAATTCTCCAAAATGGCCCGAGACCCCCAGCGCTACCTTGTCATTCAGGGGGATGAAAGA
ATGCATTTGCCAAGTCCTACAGACTCCAACTTCTACCGTGCCCTGATGGATGAAGAAGACATGGACGACG
TGGTGGATGCCGACGAGTACCTCATCCCACAGCAGGGCTTCTTCAGCAGCCCCTCCACGTCACGGACTCC
CCTCCTGAGCTCTCTGAGTGCAACCAGCAACAATTCCACCGTGGCTTGCATTGATAGAAATGGGCTGCAA
AGCTGTCCCATCAAGGAAGACAGCTTCTTGCAGCGATACAGCTCAGACCCCACAGGCGCCTTGACTGAGG
ACAGCATAGACGACACCTTCCTCCCAGTGCCTGGTGAGTGGCTTGTCTGGAAACAGTCCTGCTCCTCAAC
CTCCTCGACCCACTCAGCAGCAGCCAGTCTCCAGTGTCCAAGCCAGGTGCTCCCTCCAGCATCTCCAGAG
GGGGAAACAGTGGCAGATTTGCAGACACAGTGAAGGGCGTAAGGAGCAGATAAACACATGACCGAGCCTG
CACAAGCTCTTTGTTGTGTCTGGTTGTTTGCTGTACCTCTGTTGTAAGAATGAATCTGCAAAATTTCTAG
CTTATGAAGCAAATCACGGACATACACATCTGTATGTGTGAGTGTTCATGATGTGTGTACATCTGTGTAT
GTGTGTGTGTGTATGTGTGTGTTTGTGACAGATTTGATCCCTGTTCTCTCTGCTGGCTCTATCTTGACCT
GTGAAACGTATATTTAACTAATTAAATATTAGTTAATATTAATAAATTTTAAGCTTTATCCAGAAAAAAA
AAAAAAAAA

A wild-type EGFR protein may be encoded by the following human amino acid sequence (GenBank Accession No. AAH94761.1) (SEQ ID NO: 2):

MRPSGTAGAALLALLAALCPASRALEEKKVCQGTSNKLTQLGTFEDHFLSLQRMFNNCEVVLGNLEITY
VQRNYDLSFLKTIQEVAGYVLIALNTVERIPLENLQIIRGNMYYENSYALAVLSNYDANKTGLKELPMR
NLQGQKCDPSCPNGSCWGAGEENCQKLTKIICAQQCSGRCRGKSPSDCCHNQCAAGCTGPRESDCLVCR
KFRDEATCKDTCPPLMLYNPTTYQMDVNPEGKYSFGATCVKKCPRNYVVTDHGSCVRACGADSYEMEED
GVRKCKKCEGPCRKVCNGIGIGEFKDSLSINATNIKHFKNCTSISGDLHILPVAFRGDSFTHTPPLDPQ
ELDILKTVKEITGFLLIQAWPENRTDLHAFENLEIIRGRTKQHGQFSLAVVSLNITSLGLRSLKEISDG
DVIISGNKNLCYANTINWKKLFGTSGQKTKIISNRGENSCKATGQVCHALCSPEGCWGPEPRDCVSCRN
VSRGRECVDKCNLLEGEPREFVENSECIQCHPECLPQAMNITCTGRGPDNCIQCAHYIDGPHCVKTCPA
GVMGENNTLVWKYADAGHVCHLCHPNCTYGCTGPGLEGCPTNGPKIPSIATGMVGALLLLLVVALGIGL
FMRRRHIVRKRTLRRLLQERELVEPLTPSGEAPNQALLRILKETEFKKIKVLGSGAFGTVYKGLWIPEG
EKVKIPVAIKELREATSPKANKEILDEAYVMASVDNPHVCRLLGICLTSTVQLITQLMPFGCLLDYVRE
HKDNIGSQYLLNWCVQIAKGMNYLEDRRLVHRDLAARNVLVKTPQHVKITDFGLAKLLGAEEKEYHAEG
GKVPIKWMALESILHRIYTHQSDVWSYGVTVWELMTFGSKPYDGIPASEISSILEKGERLPQPPICTID
VYMIMVKCWMIDADSRPKFRELIIEFSKMARDPQRYLVIQGDERMHLPSPTDSNFYRALMDEEDMDDVV
DADEYLIPQQGFFSSPSTSRTPLLSSLSATSNNSTVACIDRNGLQSCPIKEDSFLQRYSSDPTGALTED
SIDDTFLPVPGEWLVWKQSCSSTSSTHSAAASLQCPSQVLPPASPEGETVADLQTQ

Multiple precursor isoforms and alternatively spliced transcript variants that encode different EGFR variants have been identified. The nucleic acid and polypeptide sequences of multiple precursor isoforms of EGFR are known in the art: for example, isoform A (GenBank Accession Number NM_005228.3 and NP 005219.2), isoform B (NM_201282.1 and NP_958439.1), isoform C (NM_201283.1 and NP_58440.1) and isoform D (NM_201284.1 and NP_958441.1). The present disclosure features an EGFR variant that has a deletion that begins within exon 3 to within exon 7, thereby deleting part of exon 3, all of exons 4-6, and part of exon 7. The deletion is preferably a deletion of amino acids 90-221 of wild-type EGFR.

EGFR and other HER receptors are receptor tyrosine kinases, and are activated though binding of its specific ligands. EGFR ligands are known in the art and include growth factors such as EGF (epidermal growth factor), TGF-α (transforming growth factor alpha), HB-EGF (heparin-binding EGF-like growth factor), amphiregulin (AR), betacellulin (BTC), epigen and epiregulin (EPR). Upon binding of one of its ligands, EGFR is activated to form a homodimer with another EGFR monomer. In some instances, EGFR may also pair with another member of the ErbB receptor family to form an active heterodimer. EGFR dimerization stimulates its intrinsic intracellular protein tyrosine kinase activity, resulting in autophosphoryaltion of several tyrosine (Y) residues in the C-terminal domain of EGFR. The tyrosines that can be phosphorylated include Y992, Y1045, Y1069, Y1148 and Y1173. Tyrosine autophosphorylation activates downstream signaling by several other proteins that are associated with the phosphorylated tyrosines of EGFR through phosphor-tyrosine-binding SH2 domains. Such downstream signaling can activate several signal transduction cascades, principally the MAPK, Akt and JNK pathways. These signaling cascades are known to play essential roles in DNA synthesis, cell proliferation and cell growth, as well as modulation of cellular phenotypes, such as cell migration and adhesion.

EGFR and other components of the HER signaling pathway interact in a complex and tightly regulated manner to regulate cell growth. Alterations in the amount or activity of HER family members, for example, EGFR, may cause or support the inappropriate cell growth that leads to proliferation, migration, and survival of cancer cells. Because the signaling pathway works as a cascade that amplifies the growth signal at each step, small changes in the amount or activity of EGFR may significantly drive the development, or progression, of cancer by promoting cell growth and metastasis, (e.g., cell migration) and inhibiting apoptosis (programmed cell death).

Mutations and overexpression of EGFR have been implicated as important factors in the proliferation of malignancies and have been utilized as markers of poor prognosis for certain cancers. EGFR was the first cell surface glycoprotein identified to be amplified and rearranged in glioblastoma multiforme (GBM) and to act oncogenically to stimulate the growth and spread of cancer cells. EGFR has also been shown to play a role in oncogenesis and progression of other cancers, such as glioblastoma, astrocytoma, meningioma, head and neck squamous cell cancer, melanoma, cervical cancer, renal cell cancer, lung cancer, prostate cancer, bladder cancer, colorectal cancer, pancreatic cancer and breast cancer.

### Identification of a novel EGFR variant

Many different variants, also referred to herein as mutants, of EGFR have been identified, and many of these EGFR variants have been implicated in the initiation or progression of certain cancers. Mutations of EGFR are commonly small deletions in the N or C terminus. EGFRvIII, which is an in-frame deletion corresponding to exons 2-7 in the mRNA, is presently known as the most common EGFR variant and was first identified in glioblastoma multiforme (GBM).

EGFR variants known in the art include: EGFRvI (N-terminal truncation), EGFRvII (deletion of exons 14-15), EGFRvIII (deletion of exons 2-7), EGFRvIII/Δ12-13 (deletion of exons 2-7 and exons 12-13), EGFRvIV (deletion of exons 25-27), EGFRvV (C-terminal truncation, EGFR.TDM/2-7 (tandem duplications of exons 2-7), EGFR.TDM/18-25 (tandem duplications of exons 18-25) and EGFR.TDM/18-26 (tandem duplications of exons 18-26).

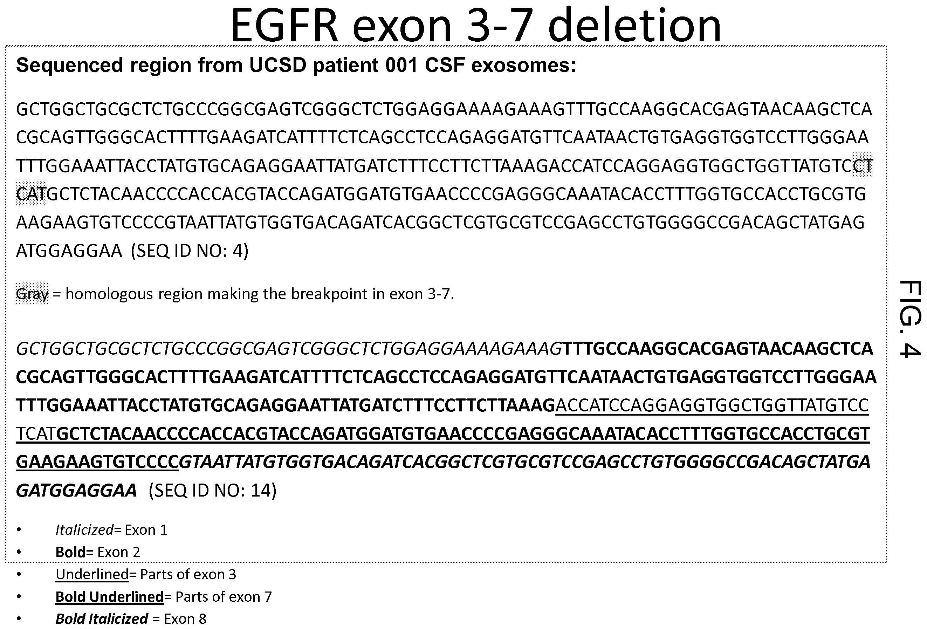

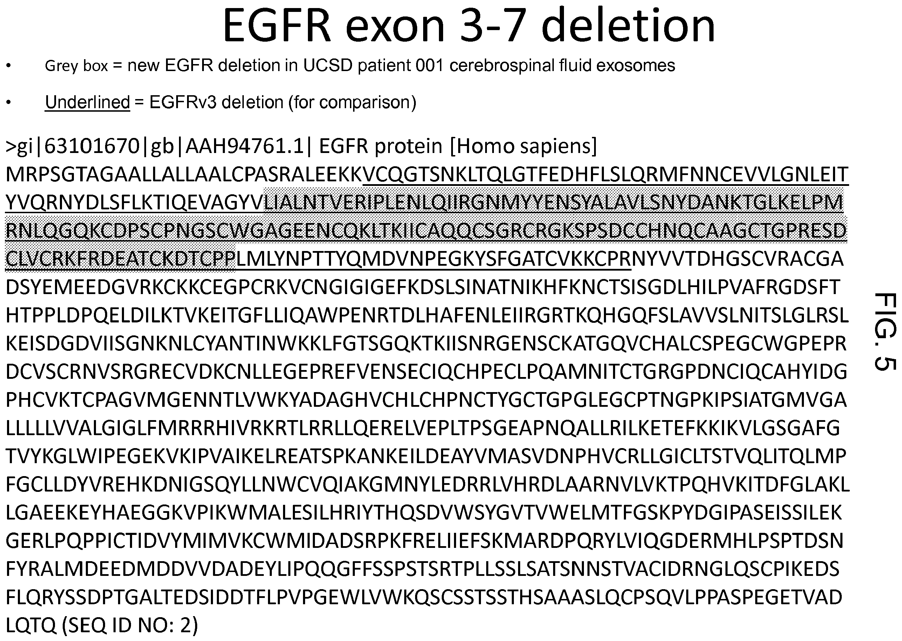

The present disclosure features a novel EGFR variant, referred to herein as EGFRvVI. The novel EGFR variant was identified as described in Example 1. EGFRvVI contains an in-frame deletion that corresponds to part of exon 3 through part of exon 7. Specifically, the deletion begins within exon 3 and ends within exon 7. Preferably, the deletion begins one third into exon 3 and ends halfway into exon 7. The homologous region constituting the breakpoint within exon 3 and exon 7 that causes the deletion is depicted in Figure 4. The deletion is from amino acids 90-221 of the wild-type EGFR (e.g., SEQ ID NO: 2), and results in an EGFR variant that is 959 amino acids long (e.g., SEQ ID NO: 5), as shown in Figure 5. Preferably, the deletion is 10730 base pairs (bp) long. The variant may have 1, 2, 3, 4, 5, 10, 15 or 20 additional amino acids deleted from the sequences N-terminal to amino acid at position 90 or C-terminal to the amino acid at position 221 of the wild-type EGFR. Conversely, the variant may have a 1, 2, 3, 4, 5, 10, 15, or 20 additional amino acids C-terminal to amino acid position 90 or N-terminal to the amino acid at position 221, wherein the additional amino acids are identical to the amino acids at the corresponding positions of the wild-type EGFR. The deletions described herein may be present in any of the splice variants or isoforms of EGFR.

The EGFR variant may comprise a nucleotide sequence greater than 60%, 65%, 70%, 75%, 80%, 85%, 90%, 91%, 92%, 93%, 94%, 95%, 96%, 97%, 98%, or 99% identity to the nucleotide sequence of the EGFR variant, e.g., SEQ ID NO: 1. The EGFR variant may comprise an amino acid sequence greater than 90% identity to the amino acid sequence of the EGFR variant, e.g., SEQ ID NO: 5.

The term"% identity," in the context of two or more nucleotide or amino acid sequences, refer to two or more sequences or subsequences that are the same or have a specified percentage of amino acid residues or nucleotides that are the same, when compared and aligned for maximum correspondence, as measured using one of the following sequence comparison algorithms or by visual inspection. For example, % identity is relative to the entire length of the coding regions of the sequences being compared.

For sequence comparison, typically one sequence acts as a reference sequence, to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters. Percent identity can be determined using search algorithms such as BLAST and PSI-BLAST (Altschul et al., 1990, J Mol Biol 215:3, 403-410; Altschul et al., 1997, Nucleic Acids Res 25:17, 3389-402).

The present disclosure features a novel EGFR variant, EGFRvVI, comprising the nucleotide sequence shown below. The underlined sequence indicates the homologous region that constitutes the breakpoint within exons 3 and 7.

GTCCGGGCAGCCCCCGGCGCAGCGCGGCCGCAGCAGCCTCCTCCCCCCGCACGGTGTGAGCGCCCGCCG
CGGCCGAGGCGGCCGGAGTCCCGAGCTAGCCCCGGCGGCCGCCGCCGCCCAGACCGGACGACAGGCCA
CCTCGTCGGCGTCCGCCCGAGTCCCCGCCTCGCCGCCAACGCCACAACCACCGCGCACGGCCCCCTGACT
CCGTCCAGTATTGATCGGGAGAGCCGGAGCGAGCTCTTCGGGGAGCAGCGATGCGACCCTCCGGGACGG
CCGGGGCAGCGCTCCTGGCGCTGCTGGCTGCGCTCTGCCCGGCGAGTCGGGCTCTGGAGGAAAAGAAAG
TTTGCCAAGGCACGAGTAACAAGCTCACGCAGTTGGGCACTTTTGAAGATCATTTTCTCAGCCTCCAGAG
GATGTTCAATAACTGTGAGGTGGTCCTTGGGAATTTGGAAATTACCTATGTGCAGAGGAATTATGATCTT
TCCTTCTTAAAGACCATCCAGGAGGTGGCTGGTTATGTCCTCATGCTCTACAACCCCACCACGTACCAGA
TGGATGTGAACCCCGAGGGCAAATACACCTTTGGTGCCACCTGCGTGAAGAAGTGTCCCCGTAATTATGT
GGTGACAGATCACGGCTCGTGCGTCCGAGCCTGTGGGGCCGACAGCTATGAGATGGAGGAAGACGGCGT
CCGCAAGTGTAAGAAGTGCGAAGGGCCTTGCCGCAAAGTGTGTAACGGAATAGGTATTGGTGAATTTAA
AGACTCACTCTCCATAAATGCTACGAATATTAAACACTTCAAAAACTGCACCTCCATCAGTGGCG

ATCTCCACATCCTGCCGGTGGCATTTAGGGGTGACTCCTTCACACATACTCCTCCTCTGGATCCACAGG
AACTGGATATTCTGAAAACCGTAAAGGAAATCACAGGGTTTTTGCTGATTCAGGCTTGGCCTGAAAACA
GGACGGACCTCCATGCCTTTGAGAACCTAGAAATCATACGCGGCAGGACCAAGCAACATGGTCAGTTTT
CTCTTGCAGTCGTCAGCCTGAACATAACATCCTTGGGATTACGCTCCCTCAAGGAGATAAGTGATGGAG
ATGTGATAATTTCAGGAAACAAAAATTTGTGCTATGCAAATACAATAAACTGGAAAAAACTGTTTGGGA
CCTCCGGTCAGAAAACCAAAATTATAAGCAACAGAGGTGAAAACAGCTGCAAGGCCACAGGCCAGGTCT
GCCATGCCTTGTGCTCCCCCGAGGGCTGCTGGGGCCCGGAGCCCAGGGACTGCGTCTCTTGCCGGAATG
TCAGCCGAGGCAGGGAATGCGTGGACAAGTGCAACCTTCTGGAGGGTGAGCCAAGGGAGTTTGTGGAGA
ACTCTGAGTGCATACAGTGCCACCCAGAGTGCCTGCCTCAGGCCATGAACATCACCTGCACAGGACGGG
GACCAGACAACTGTATCCAGTGTGCCCACTACATTGACGGCCCCCACTGCGTCAAGACCTGCCCGGCAG
GAGTCATGGGAGAAAACAACACCCTGGTCTGGAAGTACGCAGACGCCGGCCATGTGTGCCACCTGTGCC
ATCCAAACTGCACCTACGGATGCACTGGGCCAGGTCTTGAAGGCTGTCCAACGAATGGGCCTAAGATCC
CGTCCATCGCCACTGGGATGGTGGGGGCCCTCCTCTTGCTGCTGGTGGTGGCCCTGGGGATCGGCCTCT
TCATGCGAAGGCGCCACATCGTTCGGAAGCGCACGCTGCGGAGGCTGCTGCAGGAGAGGGAGCTTGTGG
AGCCTCTTACACCCAGTGGAGAAGCTCCCAACCAAGCTCTCTTGAGGATCTTGAAGGAAACTGAATTCA
AAAAGATCAAAGTGCTGGGCTCCGGTGCGTTCGGCACGGTGTATAAGGGACTCTGGATCCCAGAAGGTG
AGAAAGTTAAAATTCCCGTCGCTATCAAGGAATTAAGAGAAGCAACATCTCCGAAAGCCAACAAGGAAA
TCCTCGATGAAGCCTACGTGATGGCCAGCGTGGACAACCCCCACGTGTGCCGCCTGCTGGGCATCTGCC
TCACCTCCACCGTGCAGCTCATCACGCAGCTCATGCCCTTCGGCTGCCTCCTGGACTATGTCCGGGAAC
ACAAAGACAATATTGGCTCCCAGTACCTGCTCAACTGGTGTGTGCAGATCGCAAAGGGCATGAACTACT
TGGAGGACCGTCGCTTGGTGCACCGCGACCTGGCAGCCAGGAACGTACTGGTGAAAACACCGCAGCATG
TCAAGATCACAGATTTTGGGCTGGCCAAACTGCTGGGTGCGGAAGAGAAAGAATACCATGCAGAAGGAG
GCAAAGTGCCTATCAAGTGGATGGCATTGGAATCAATTTTACACAGAATCTATACCCACCAGAGTGATG
TCTGGAGCTACGGGGTGACCGTTTGGGAGTTGATGACCTTTGGATCCAAGCCATATGACGGAATCCCTG
CCAGCGAGATCTCCTCCATCCTGGAGAAAGGAGAACGCCTCCCTCAGCCACCCATATGTACCATCGATG
TCTACATGATCATGGTCAAGTGCTGGATGATAGACGCAGATAGTCGCCCAAAGTTCCGTGAGTTGATCA
TCGAATTCTCCAAAATGGCCCGAGACCCCCAGCGCTACCTTGTCATTCAGGGGGATGAAAGAATGCATT
TGCCAAGTCCTACAGACTCCAACTTCTACCGTGCCCTGATGGATGAAGAAGACATGGACGACGTGGTGG
ATGCCGACGAGTACCTCATCCCACAGCAGGGCTTCTTCAGCAGCCCCTCCACGTCACGGACTCCCCTCC
TGAGCTCTCTGAGTGCAACCAGCAACAATTCCACCGTGGCTTGCATTGATAGAAATGGGCTGCAAAGCT
GTCCCATCAAGGAAGACAGCTTCTTGCAGCGATACAGCTCAGACCCCACAGGCGCCTTGACTGAGGACA
GCATAGACGACACCTTCCTCCCAGTGCCTGGTGAGTGGCTTGTCTGGAAACAGTCCTGCTCCTCAACCT
CCTCGACCCACTCAGCAGCAGCCAGTCTCCAGTGTCCAAGCCAGGTGCTCCCTCCAGCATCTCCAGAGG
GGGAAACAGTGGCAGATTTGCAGACACAGTGAAGGGCGTAAGGAGCAGATAAACACATGACCGAGCCTG
CACAAGCTCTTTGTTGTGTCTGGTTGTTTGCTGTACCTCTGTTGTAAGAATGAATCTGCAAAATTTCTA
GCTTATGAAGCAAATCACGGACATACACATCTGTATGTGTGAGTGTTCATGATGTGTGTACATCTGTGT
ATGTGTGTGTGTGTATGTGTGTGTTTGTGACAGATTTGATCCCTGTTCTCTCTGCTGGCTCTATCTTGA
CCTGTGAAACGTATATTTAACTAATTAAATATTAGTTAATATTAATAAATTTTAAGCTTTATCCAGAAA
AAAAAAAAAAAAA (SEQ ID NO: 3)

The present disclosure features a novel EGFR variant, EGFRvVI, comprising the nucleotide sequence containing a portion of EGFRvVI, specifically exons 1, 2, 3, 7 and 8 as follows, wherein the underlined sequence indicates the homologous region that constitutes the breakpoint within exons 3-7:

GCTGGCTGCGCTCTGCCCGGCGAGTCGGGCTCTGGAGGAAAAGAAAGTTTGCCAAGGCACGAGTAACA
AGCTCACGCAGTTGGGCACTTTTGAAGATCATTTTCTCAGCCTCCAGAGGATGTTCAATAACTGTGAGGT
GGTCCTTGGGAATTTGGAAATTACCTATGTGCAGAGGAATTATGATCTTTCCTTCTTAAAGACCATCCAG
GAGGTGGCTGGTTATGTCCTCATGCTCTACAACCCCACCACGTACCAGATGGATGTGAACCCCGAGGGC
AAATACACCTTTGGTGCCACCTGCGTGAAGAAGTGTCCCCGTAATTATGTGGTGACAGATCACGGCTC
GTGCGTCCGAGCCTGTGGGGCCGACAGCTATGAGATGGAGGAA (SEQ ID NO: 4)

The present disclosure features a novel EGFR variant, EGFRvVI, with the amino acid sequence as follows:

MRPSGTAGAALLALLAALCPASRALEEKKVCQGTSNKLTQLGTFEDHFLSLQRMFNNCEVVLGNLEITYVQR
NYDLSFLKTIQEVAGYVLMLYNPTTYQMDVNPEGKYSFGATCVKKCPRNYVVTDHGSCVRACGADSYEMEE
DGVRKCKKCEGPCRKVCNGIGIGEFKDSLSINATNIKHFKNCTSISGDLHILPVAFRGDSFTHTPPLDPQELDILK
TVKEITGFLLIQAWPENRTDLHAFENLEIIRGRTKQHGQFSLAVVSLNITSLGLRSLKEISDGDVIISGNKNLCYA
NTINWKKLFGTSGQKTKIISNRGENSCKATGQVCHALCSPEGCWGPEPRDCVSCRNVSRGRECVDKCNLLEGE
PREFVENSECIQCHPECLPQAMNITCTGRGPDNCIQCAHYIDGPHCVKTCPAGVMGENNTLVWKYADAGHVC
HLCHPNCTYGCTGPGLEGCPTNGPKIPSIATGMVGALLLLLVVALGIGLFMRRRHIVRKRTLRRLLQERELVEP
LTPSGEAPNQALLRILKETEFKKIKVLGSGAFGTVYKGLWIPEGEKVKIPVAIKELREATSPKANKEILDEAYVM
ASVDNPHVCRLLGICLTSTVQLITQLMPFGCLLDYVREHKDNIGSQYLLNWCVQIAKGMNYLEDRRLVHRDL
AARNVLVKTPQHVKITDFGLAKLLGAEEKEYHAEGGKVPIKWMALESILHRIYTHQSDVWSYGVTVWELMTF
GSKPYDGIPASEISSILEKGERLPQPPICTIDVYMIMVKCWMIDADSRPKFRELIIEFSKMARDPQRYLVIQGDER
MHLPSPTDSNFYRALMDEEDMDDVVDADEYLIPQQGFFSSPSTSRTPLLSSLSATSNNSTVACIDRNGLQSCPIK
EDSFLQRYSSDPTGALTEDSIDDTFLPVPGEWLVWKQSCSSTSSTHSAAASLQCPSQVLPPASPEGETVADLQT
Q (SEQ ID NO: 5)

EGFRvVI may be preferentially expressed in tumor cells. Exemplary tumor cells include glioblastoma cells. Other tumor cells can include epithelial tumor cells. Tumor cells can include, but are not limited to tumor cells of the following cancers: glioblastoma, astrocytoma, meningioma, head and neck squamous cell cancer, melanoma, cervical cancer, renal cell cancer, lung cancer, prostate cancer, bladder cancer, colorectal cancer, pancreatic cancer and breast cancer.

Identification of EGFRvVI in a patient may be used to determine, whether the patient has cancer, is at risk for developing cancer, or has a higher predisposition for developing cancer. This is due in part to the fact that EGFR variants are not expressed in healthy subjects that do not have cancer. Certain EGFR variants may be more prevalent in certain cancers. For example, EGFRvIII is the most frequent EGFR mutation found in non-small cell lung carcinoma (NSCLC) and GBM. EGFRvVI may be found in patients that have a neurologic cancer, for example, GBM or astrocytoma.

Cancer, as used herein, refers to a hyperproliferative condition or disorder. The term encompasses malignant as well as non-malignant cell populations. Such disorders are characterized by increased or excessive cell proliferation of one or more subsets of cells, which often appear to differ from the surrounding tissue both morphologically and genotypically. The increased or excessive cell proliferation in a tissue sample from a patient can be determined by comparison of cell proliferation from the tissue sample to a reference sample. The reference sample can be a score determined from a population of samples from a general population or a population of healthy subjects or subjects that have not been diagnosed with cancer. The reference sample can also be a sample from the same patient obtained at a different timepoint. Cell proliferation can be measured by any of the methods known in the art, such as immunohistochemical staining of biological samples for proliferation markers, such as Ki-67, BrDU, or mitotic markers. Hyperproliferative cell disorders can occur in different types of animals and in humans, and produce different physical manifestations depending upon the affected cells.

Cancers include neurologic cancers, epithelial cancers and solid tumor cancers. Such cancers include glioblastoma, astrocytoma, meningioma, head and neck squamous cell cancer, melanoma, cervical cancer, renal cell cancer, lung cancer, prostate cancer, bladder cancer, colorectal cancer, pancreatic cancer and breast cancer.

Cancers of particular interest are neurologic cancers, including brain tumors. Neurologic tumors are classified according to the kind of cell from which the tumor seems to originate, e.g., astrocytes. Diffuse, fibrillary astrocytomas are the most common type of primary brain tumor in adults. These tumors are divided histopathologically into three grades of malignancy: World Health Organization (WHO) grade II astrocytoma, WHO grade III anaplastic astrocytoma and WHO grade N glioblastoma multiforme (GBM). WHO grade II astrocytomas are the most indolent of the diffuse astrocytoma spectrum. Astrocytomas display a remarkable tendency to infiltrate the surrounding brain, confounding therapeutic attempts at local control. These invasive abilities are often apparent in low-grade as well as high-grade tumors.

Glioblastoma multiforme is the most malignant stage of astrocytoma, with survival times of less than 2 years for most patients. Histologically, these tumors are characterized by high proliferation indices, endothelial proliferation and focal necrosis. The highly proliferative nature of these lesions likely results from multiple mitogenic effects. One of the hallmarks of GBM is endothelial proliferation. A host of growth factors and their receptors are found amplified and/or mutated in GBMs.

### Antibodies

The EGFR variant, including fragments, derivatives and analogs thereof, may be used as an immunogen to produce antibodies having use in the diagnostic, research, and therapeutic methods described below. The antibodies may be polyclonal or monoclonal, chimeric, humanized, single chain (svFc) or Fab fragments. Various procedures known to those of ordinary skill in the art may be used for the production and labeling of such antibodies and fragments. See, e.g., Bums, ed., Immunochemical Protocols, 3.sup.rd ed., Humana Press (2005); Harlow and Lane, Antibodies: A Laboratory Manual, Cold Spring Harbor Laboratory (1988); Kozbor et al., Immunology Today 4: 72 (1983); Kohler and Milstein, Nature 256: 495 (1975).

Antibodies or fragments exploiting the differences between EGFRvVI and full length wild-type EGFR or other EGFR variants are particularly preferred. Of particular interest are antibodies or functional fragments thereof that specifically recognize EGFRvVI. What is meant by "specifically recognize" is that the antibodies or functional fragments thereof preferentially bind to EGFRvVI compared to wild-type EGFR and/or other EGFR variants, for example, EGFRvIII. For example, the antibody may bind to the region of EGFRvVI in which exons 3 and 7 are fused. Alternatively, the three-dimensional, or native, conformation of the native EGFRvVI protein may present a novel epitope that may not exist or may not accessible in wild-type EGFR or other EGFR variants, which can be recognized by the EGFRvVI-specific antibodies disclosed herein.

Exemplary uses for the EGFRvVI antibody are disclosed herein. Such uses include diagnostic and prognostic methods.

### Diagnostic Applications

The present disclosure provides DNA, RNA and protein based diagnostic methods that either directly or indirectly detect EGFRvVI. The present disclosure also provides compositions and kits for diagnostic purposes.

The present disclosure features a diagnostic method comprising obtaining a biological sample from a patient, extracting nucleic acids or proteins, and determining the presence, absence, or level of EGFRvVI in the biological sample. Methods for determining the presence, absence, or level of EGFRvVI in a biological sample are discussed further herein.

The diagnostic methods of the present disclosure may be qualitative or quantitative. Quantitative diagnostic methods may be used, for example, to discriminate between indolent and aggressive cancers via a predetermined cutoff or threshold level. Where applicable, qualitative or quantitative diagnostic methods may also include amplification of target, signal or intermediary (e.g., a universal primer).

EGFRvVI may be detected along with other markers in a multiplex or panel format. Markers are selected for their predictive value alone or in combination with the EGFR variant. Such markers may also include one or more of the other EGFR variants, such as EGFRvIII. Markers for other cancers, diseases, infections, and metabolic conditions known in the art are also contemplated for inclusion in a multiplex of panel format.

The diagnostic methods may also include consideration to data correlating particular EGFR variants, such as EGFRvVI, with the stage, aggressiveness or progression of the disease or the presence or risk of metastasis. Ultimately, the information provided by the methods of the present disclosure will assist a physician in choosing the best course of treatment for a particular patient.

### Samples

As used herein, the term "biological sample" refers to a sample that contains biological materials such as a DNA, an RNA and a protein. The biological sample may be a tissue sample, a cell culture sample, or a biopsy sample. The biological sample may suitably comprise a bodily fluid from a subject. The bodily fluids can be fluids isolated from anywhere in the body of the subject, preferably a peripheral location, including but not limited to, for example, blood, plasma, serum, urine, sputum, spinal fluid, cerebrospinal fluid, pleural fluid, nipple aspirates, lymph fluid, fluid of the respiratory, intestinal, and genitourinary tracts, tear fluid, saliva, breast milk, fluid from the lymphatic system, semen, cerebrospinal fluid, intra-organ system fluid, ascitic fluid, tumor cyst fluid, amniotic fluid and combinations thereof. The preferred body fluid for use as the biological sample may be urine. The preferred body fluid may be cerebrospinal fluid (CSF), serum or plasma. Suitably, a sample volume of about 0.1 ml to about 30 ml fluid may be used. The volume of fluid may depend on a few factors, e.g., the type of fluid used. For example, the volume of serum samples may be about 0.1 ml to about 20 ml, preferably about 4 ml. The volume of urine samples may be about 10 ml to about 30 ml, preferably about 20ml.

As described herein, the sample may be in vivo, in which circulating tumor cells or microvesicles (e.g., exosomes) that express EGFRvVI can be detected from biofluids in vivo via insertion of a venous catheter. Specifically, the venous catheter comprises a detection agent that can bind to EGFRvVI, e.g., an EGFRvVI-specific antibody. The catheter then detects or collects the EGFRvVI-expressing circulating tumor cells or microvesicles. The venous catheter can be left inserted into the subject for at least 5 minutes, to survey at least 2.5 liters of blood. Thus, this method allows for detection of rare tumor events.

The term "subject" is intended to include all animals shown to or expected to express EGFR and variants thereof. The subject may be a mammal, a human or nonhuman primate, a dog, a cat, a horse, a cow, other farm animals, or a rodent (e.g. mice, rats, guinea pig. etc.). A human subject may be a normal human being without observable abnormalities, e.g., a disease. A human subject may be a human being with observable abnormalities, e.g., a disease. The observable abnormalities may be observed by the human being himself, or by a medical professional. The term "subject", "patient", and "individual" are used interchangeably herein.

The biological sample may require preliminary processing designed to isolate or enrich the sample for EGFRvVI or other markers of interest. Cells or other nucleic acid-containing particles that contain EGFRvVI or other markers of interest may be isolated. A variety of techniques known to those of ordinary skill in the art may be used for this purpose, including but not limited: centrifugation; immunocapture; cell lysis; and nucleic acid target capture (See, e.g., EP Pat. No. 1409 727).

It may be advantageous to pre-process the biological sample such that a microvesicle fraction is isolated, purified or enriched. As used herein, the
term "microvesicles" refers collectively to all membrane vesicles less than 0.8 microns that are shed by a cell. Isolation of microvesicles from a biological sample can be achieved through centrifugation, filtration, ion exchange chromatography, size exclusion chromatography and affinity chromatography, or any combination thereof (*See,* e.g., PCT Applications WO2011/009104, WO0012/051622, and WO2012/064993). Isolation of the microvesicle fraction prior to extraction of nucleic acids or proteins results in higher quality extractions for more accurate or specific detection of EGFRvVI and other markers of interest.

### Nucleic Acid Detection

EGFRvVI may be detected as chromosomal rearrangements of genomic DNA or chimeric mRNA using a variety of nucleic acid techniques known to those of ordinary skill in the art, including but not limited to: nucleic acid sequencing; nucleic acid hybridization; and nucleic acid amplification.

Examples of nucleic acid sequencing techniques include, but are not limited to, chain terminator (Sanger) sequencing and dye terminator sequencing. Those of ordinary skill in the art will recognize that because RNA is less stable in the cell and more prone to nuclease attack, RNA is usually reverse transcribed to DNA before sequencing. Methods and reagents for reverse-transcribing RNA to cDNA are known in the art. Chain terminator sequencing uses sequence-specific termination of a DNA synthesis reaction using modified nucleotide substrates, radioactive or other labeled oligonucleotide primers, and chain terminating nucleotides. Dye terminator sequencing labels the terminators and complete sequencing can be performed in a single reaction by labeling each di-deoxynucleotide chain terminator with a separate fluorescent dye.

Examples of nucleic acid hybridization techniques include, but are not limited to, in situ hybridization (ISH, such as fluorescent in situ hybridization FISH), micro array, and Southern or Northern blot. ISH uses a labeled complementary DNA or RNA probes to localize, or hybridize, to a specific DNA or RNA sequence in a portion or section of tissue (in situ). Examples of microarrays include DNA microarrays (e.g., cDNA microarrays and oligonucleotide microarrays), protein microarrays; tissue microarrays, transfection or cell microarrays, chemical compound microarrays, and antibody microarrays. A DNA microarray is also commonly known as a gene chip, DNA chip, or biochip. Microarrays are a collection of probes (e.g., DNA, RNA, compounds or antibodies) attached to a solid surface (e.g., glass, plastic or silicon chip) forming an array for the purpose of monitoring expression profiles of thousands of genes or proteins simultaneously. Southern blotting is utilized to detect specific filter-bound DNA sequences by hybridization with a labeled probe complementary to the sequence of interest. Northern blotting is utilized to detect specific filter-bound RNA sequences by hybridization with a labeled probe complementary to the sequence of interest.

EGFRvVI can be identified by amplification which is performed either prior to or simultaneously with detection. Examples of nucleic acid amplification techniques include, but are not limited to, polymerase chain reaction (PCR), reverse transcription polymerase chain reaction (RT-PCR), transcription-mediated amplification (TMA), ligase chain reaction (LCR), strand displacement amplification (SDA), and nucleic acid sequence based amplification (NASBA). Those of ordinary skill in the art will recognize that certain amplification techniques (e.g., PCR) require that RNA be reversed transcribed to DNA prior to amplification (e.g., RT-PCR), whereas other amplification techniques directly amplify RNA (e.g., TMA and NASBA).

The polymerase chain reaction (U.S. Pat. Nos. 4,683,195, 4,683,202, 4,800,159 and 4,965,188), commonly referred to as PCR, uses multiple cycles of denaturation, annealing of primer pairs to opposite strands, and primer extension to exponentially increase copy numbers of a target nucleic acid sequence. In a variation called RT-PCR, reverse transcriptase (RT) is used to make a complementary DNA (cDNA) from mRNA, and the cDNA is then amplified by PCR to produce multiple copies of DNA. For other various permutations of PCR see, e.g., U.S. Pat. Nos. 4,683,195, 4,683,202 and 4,800,159; Mullis et al., Meth. Enzymol. 155: 335 (1987); and, Murakawa et al., DNA 7: 287 (1988). Transcription-mediated amplification, commonly referred to as TMA, synthesizes multiple copies of a target nucleic acid sequence autocatalytically under conditions of substantially constant temperature, ionic strength, and pH in which multiple RNA copies of the target sequence autocatalytically generate additional copies. The ligase chain reaction, commonly referred to as LCR, uses two sets of complementary DNA oligonucleotides that hybridize to adjacent regions of the target nucleic acid. The DNA oligonucleotides are covalently linked by a DNA ligase in repeated cycles of thermal denaturation, hybridization and ligation to produce a detectable double-stranded and ligated oligonucleotide product. Strand displacement amplification, commonly referred to as SDA, uses cycles of annealing pairs of primer sequences to opposite strands of a target sequence, primer extension in the presence of dNTPaS to produce a duplex hemiphosphorothioated primer extension product. This primer extension product undergoes endonuclease-mediated nicking at a hemimodified restriction endonuclease recognition site, and polymerase-mediated primer extension from the 3' end of the nick displaces an existing strand and produces a strand for the next round of primer annealing, nicking and strand displacement, thereby resulting in geometric amplification of product.

Non-amplified or amplified EGFR variant nucleic acids can be detected by any conventional means. For example, EGFRvVI can be detected by hybridization with a detectably labeled probe and measurement of the resulting hybrids. Illustrative non-limiting examples of detection methods are described below.

Quantitative evaluation of the amplification process can be performed in real-time. Evaluation of an amplification process in "real-time" involves determining the amount of amplicon in the reaction mixture either continuously or periodically during the amplification reaction, and using the determined values to calculate the amount of target sequence initially present in the sample. A variety of methods for determining the amount of initial target sequence present in a sample based on real-time amplification are well known in the art. These include methods disclosed in U.S. Pat. Nos. 6,303,305 and 6,541,205. Another method for determining the quantity of target sequence initially present in a sample, but which is not based on a real-time amplification, is disclosed in U.S. Pat. No. 5,710,029.

The disclosure also provides nucleic acids that bind to EGFRvVI nucleic acids for the specific detection of the presence of EGFRvVI nucleic acid in a sample. The nucleic acids for detection of EGFRvVI_comprise an isolated nucleic acid consisting of 10 to 1000 nucleotides (preferably, 10 to 500, 10 to 100, 10 to 50, 10 to 35, 20 to 1000, 20 to 500, 20 to 100, 20 to 50, or 20 to 35 nucleotides) which hybridizes to RNA or DNA encoding EGFRvVI or to an EGFR gene. In a preferred aspect, the nucleic acids preferentially hybridize to RNA or DNA encoding EGFRvVI but not to RNA or DNA of wild-type EGFRvVI or other EGFR variants. Specifically, the isolated nucleic acid is or is complementary to a nucleotide sequence consisting of at least 10 consecutive nucleotides
(preferably, 15, 18, 20, 25, or 30) from the nucleic acid molecule comprising a polynucleotide sequence at least 60%, 65%, 70%, 75%, 80%, 85%, 90%, 91 %, 92%, 93%, 94%, 95%, 96%, 97%, 98%, or 99% identical to a sequence selected from the group consisting of: a nucleotide sequence encoding the EGFRvVI polypeptide comprising the amino acid sequence in SEQ ID NO:5; a nucleotide sequence comprising the nucleic acid sequence in SEQ ID NO: 3 or SEQ ID NO: 4; a nucleotide sequence of any one of exons 1-3, or exons 7-28 of EGFRvVI (SEQ ID NO:3) or wild-type EGFR, e.g., SEQ ID NO: 1, wherein the nucleotide sequence may also span adjacent exons; or a sequence complementary to any of the nucleotide sequences described above. Preferably, the nucleotide sequence does not comprise the sequence of sequence complementary to exon 4, exon 5, or exon 6 of wild-type EGFR, e.g., SEQ ID NO: 1. Complementary sequences are also known as antisense nucleic acids when they comprise sequences which are complementary to the coding strand. Preferably, the nucleic acid does not specifically hybridize to nucleotides that encode amino acids at positions 90-221 of wild-type EGFR. The nucleic acids useful for detection are also referred to herein as primers or probes and can be utilized in many of the detection methods described herein.

The isolated nucleic acid molecule for detection of EGFRvVI may comprise 10 to 50 nucleotides, which specifically hybridize to the EGFRvVI RNA, DNA or cDNA. The nucleic acid molecule is, or is complementary to, a nucleotide sequence consisting of at least 5, at least 10, at least 15, or at least 20 consecutive nucleotides from EGFRvVI exons 1-3 or exons 7-28 of EGFRvVI (SEQ ID NO: 3) or wild-type EGFR (SEQ ID NO: 1), including nucleotide sequences that span adjacent exons of exons 1-3 and 7-28. Specifically, the nucleic acid molecules do not hybridize to the nucleotide sequences that encode amino acids 90-221 of wild-type EGFR.

Examples of specific nucleic acid primers and probes which can be used in the present disclosure include:
EGFR Forw 1- CTGCTGGCTGCGCTCTG (SEQ ID NO: 6)
EGFRv3 rev4 - CGTGATCTGTCACCACATAATTACC (SEQ ID NO: 7)
EGFR probe 6 - TTCCTCCAGAGCCCGACT (SEQ ID NO: 8)
EGFR Forw 1- CTGCTGGCTGCGCTCTG (SEQ ID NO: 6)
EGFR Rev 1- TTCCTCCATCTCATAGCTGTCG (SEQ ID NO: 9)
EGFR probe 6: TTCCTCCAGAGCCCGACT (SEQ ID NO:8)
EGFR qPCR exon 3-7 del forw 1: TGGTCCTTGGGAATTTGGAA (SEQ ID NO: 10)
EGFR qPCR exon 3-7 del rev: GTGGGGTTGTAGAGCATGAGGA (SEQ ID NO: 11)
EGFR qPCR exon 3-7 del probe: TACCTATGTGCAGAGGAA (SEQ ID NO: 12)

As will be understood by the person of ordinary skill, a multitude of additional probes or primers can be designed from SEQ ID NO: 3.

The nucleic acid probe can be used to probe an appropriate chromosomal or cDNA library by usual hybridization methods to obtain another nucleic acid molecule of the present disclosure. A chromosomal DNA or cDNA library can be prepared from appropriate cells according to recognized methods in the art (cf. Molecular Cloning: A Laboratory Manual, second edition, edited by Sambrook, Fritsch, & Maniatis, Cold Spring Harbor Laboratory, 1989).

In the alternative, chemical synthesis is carried out in order to obtain nucleic acid probes having nucleotide sequences which correspond to N-terminal and C-terminal portions of the PCA3 amino acid sequence. Thus, the synthesized nucleic acid probes can be used as primers in a polymerase chain reaction (PCR) carried out in accordance with recognized PCR techniques, essentially according to PCR Protocols, A Guide to Methods and Applications, edited by Michael et al., Academic Press, 1990, utilizing the appropriate chromosomal, cDNA or cell line library to obtain the fragment of the present disclosure.

One skilled in the art can readily design such probes based on the sequence disclosed herein using methods of computer alignment and sequence analysis known in the art (cf. Molecular Cloning: A Laboratory Manual, second edition, edited by Sambrook, Fritsch, & Maniatis, Cold Spring Harbor Laboratory, 1989).

The hybridization probes of the present disclosure can be labeled by standard labeling techniques such as with a radiolabel, enzyme label, fluorescent label, biotin-avidin label, chemiluminescence, and the like. For example, the probe is labeled with FAM dye. After hybridization, the probes can be visualized using known methods.

The nucleic acid primers and probes of the present disclosure include RNA, as well as DNA probes, such probes being generated using techniques known in the art.

A nucleic acid probe may be immobilized on a solid support. Examples of such solid supports include, but are not limited to, plastics such as polycarbonate, complex carbohydrates such as agarose and sepharose, and acrylic resins, such as polyacrylamide and latex beads. Techniques for coupling nucleic acid probes to such solid supports are well known in the art.

### Protein Detection

EGFRvVI may be detected as truncated a EGFR protein, a chimeric protein or a protein with a deletion using a variety of protein techniques known to those of ordinary skill in the art, including but not limited to protein sequencing and immunoassays. Examples of protein sequencing techniques include, but are not limited to, mass spectrometry and Edman degradation. Examples of immunoassays include, but are not limited to immunoprecipitation, Western blot, ELISA, immunohistochemistry, immunocytochemistry, flow cytometry, and immuno-PCR. Polyclonal or monoclonal antibodies labeled with detectable moieties using various techniques known to those of ordinary skill in the art (e.g., calorimetric, fluorescent, chemiluminescent or radioactive labels) are suitable for use in the immunoassays.

A computer-based analysis program may be used to translate the raw data generated by the detection assay (e.g., the presence, absence, or amount of a given marker or markers, for example EGFRvVI) into data of predictive value for a clinician. The clinician can access the predictive data using any suitable means. Thus, there is provided the further benefit that the clinician, who is not likely to be trained in genetics or molecular biology, need not understand the raw data. The data is presented directly to the clinician in its most useful form. The clinician is then able to immediately utilize the information in order to optimize the care of the subject. The present disclosure contemplates any method capable of receiving, processing, and transmitting the information to and from laboratories conducting the assays, information provides, medical personal, and subjects.

EGFRvVI may also be detected using in vivo imaging techniques, including but not limited to: radionuclide imaging; positron emission tomography (PET); computerized axial tomography, X-ray or magnetic resonance imaging method, fluorescence detection, and chemiluminescent detection. In vivo imaging techniques may be used to visualize the presence of or expression of cancer markers in an animal (e.g., a human or non-human mammal). For example, cancer marker mRNA or protein may be labeled
using a labeled antibody specific for the cancer marker. A specifically bound and labeled antibody can be detected in an individual using an in vivo imaging method, including, but not limited to, radionuclide imaging, positron emission tomography, computerized axial tomography, X-ray or magnetic resonance imaging method, fluorescence detection, and chemiluminescent detection. The in vivo imaging methods of the present disclosure are useful in the diagnosis of cancers that express the cancer markers disclosed herein (e.g., glioblastoma). In vivo imaging is used to visualize the presence of a marker indicative of the cancer, e.g., EGFRvVI. Such techniques allow for diagnosis without the use of an unpleasant biopsy. The in vivo imaging methods of the present disclosuren are also useful for providing prognoses to cancer patients. For example, the presence of a marker indicative of cancers likely to metastasize can be detected. The in vivo imaging methods of the present disclosure can further be used to detect metastatic cancers in other parts of the body.

### Compositions & Kits

Compositions for use in the diagnostic methods disclosed herein include, but are not limited to, probes, amplification oligonucleotides, and antibodies. Particularly preferred compositions detect EGFRvVI, but not any other EGFR variant. These compositions include: a single labeled probe or oligonucleotide primer comprising a sequence that hybridizes to the junction at which a 5' portion from exon 3 of EGFR fuses to a 3' portion from exon 7; a pair of amplification oligonucleotides wherein the one amplification oligonucleotide comprises a sequence that hybridizes to the homologous breakpoint where exon 3 and exon 7 is joined in EGFRvVI; or an antibody that specifically recognizes EGFRvVI.

Any of these compositions, alone or in combination with other compositions of the present disclosure, may be provided in the form of a kit. For example, the single labeled probe and pair of amplification oligonucleotides may be provided in a kit for the amplification
and detection of the EGFR variant of the present disclosure. Kits may further comprise appropriate controls and/or detection reagents.

The probe and antibody compositions may also be provided in the form of an array.

### Prognostic Applications

A correlation between EGFRvVI and the prognosis of patients with cancer may exist. The presence, absence or level of the EGFRvVI may be of use for stratifying or classifying the tumor based on its activity, aggressiveness, invasiveness, or metastatic potential. Thus, assays for detecting EGFRvVI may be used to provide prognoses and help physicians decide on an appropriate therapeutic strategy. For example, patients with tumors that have EGFRvVI may be treated differently than those that have wild-type EGFR. For example, the prognosis of patients that have EGFRvVI may be worse than patients that lack EGFRvVI, and therefore may require more aggressive treatment regimens. The presence of EGFRvVI may correlate survival rate/time, invasiveness of the tumor, and metastasis.

Although the present disclosure will most preferably be used in connection with obtaining a prognosis for glioblastoma cancer patients, other epithelial or solid cell tumors may also be examined and the assays and probes described herein may be used in determining whether cancerous cells from these tumors express EGFRvVI, which is likely to make them particularly aggressive, i.e., likely to be invasive and metastatic. Examples of tumors that may be characterized using this procedure include tumors of the breast, lung, colon, ovary, uterus, esophagus, stomach, liver, kidney, brain, skin and muscle. The assays will also be of value to researchers studying these cancers in cell lines and animal models.

### Drug-Screening and Therapeutic Applications

The present disclosure provides drug-screening assays (e.g., to screen for anticancer drugs). The screening methods of the present disclosure provide for identification of compounds or agents (e.g., small molecules, drugs, proteins, peptides, peptidomimetics, peptoids) that can modulate the biological function of EGFRvVI. For example, the compounds or agents can inhibit EGFRvVI in a ligand-independent manner, as EGFRvVI lacks a portion of the ligand-binding domain. Inhibiting the biological function of EGFRvVI can include inhibiting the EGFR kinase activity, modulating signaling pathways that are upstream or downstream of EGFRvVI, or increasing proteasomal degradation of EGFRvVI. Compounds that inhibit the activity of EGFRvVI may be useful in the treatment of proliferative disorders, e.g., cancer.

It may be advantageous to modulate or inhibit EGFRvVI for treatment of proliferative disorders, such as cancer. The present disclosure encompasses therapies that target EGFRvVI directly or indirectly. Examples of therapies useful for inhibiting the function of EGFRvVI include RNA interference (RNAi) compounds such as siRNAs, and other antisense compounds that specifically hybridize to target nucleic acids encoding EGFRvVI. Other therapies that may be useful include antibodies that specifically bind and/or inhibit activity of EGFRvVI. The antibody may be conjugated to a cytotoxic agent (e.g., toxin, drug or radioactive moiety).

The present disclosure may feature methods for detecting EGFRvVI to determine or assess the responsiveness of a subject to a particular therapeutic regimen comprising analyzing the presence or absence of EGFRvVI wherein the presence or absence correlates with the responsiveness of a subject to the therapeutic regiment. The methods of detection can be used to detect EGFRvVI DNA, RNA or protein, as described herein. Preferably, the method of detection is by nucleic acid amplification, e.g., quantitative real-time PCR.

EGFR has been a popular target for molecular therapies for cancer or proliferative disorders. EGFR-specific therapies generally include inhibitors of the EGFR kinase activity and inhibitors of the ligand-mediated activation of EGFR. Examples of therapies that target EGFR include antibody inhibitors that block the extracellular N-terminal ligand binding domain. Blocking of the ligand binding domain prevents inappropriate activation and downstream signaling of EGFR. Examples of agents that block the ligand-binding domain of EGFR include cetuximab (ERBITUX®), panitumumab (VECTIBIX®), zalutumumab, nimotuzumab (BioMab EGFR), and matuzumab (EMD 7200). Other EGFR-specific inhibitors include small molecules that inhibit the EGFR tyrosine kinase activity by reversibly or irreversibly binding the ATP-binding pocket. These small molecules target the kinase domain, which is on the cytoplasmic and C-terminal end of the protein. Such inhibitors include, for example, gefitinib (IRESSA™), erlotinib (TARCEVA®) and lapatinib (TYKERB®). Other EGFR inhibitors include cancer vaccines that target EGFR deletions, such as Rindopepimut (CDX-110, Celldex Therapeutics). Other inhibitors that may be particularly useful for indirectly inhibiting EGFR and downstream signaling is PARP1 inhibitors, for example, 5-AIQ hydrochloride, ABT-888, minocycline, PARP inhibitor IX (EB-47), PARP inhibitor XI (DR2313), TIQ-A (available from Santa Cruz Biotechnology).

However, not all patients respond to treatments with these EGFR inhibitors, which can be partly attributable to the tumor-specific EGFR variants that exhibit deletions of the portions of EGFR that are targeted by certain therapeutic agents. For example, EGFRvVI lacks a significant portion of the N-terminal ligand binding domain. Presence of EGFRvVI may indicate that therapeutic regimens comprising molecules that target the ligand binding domain will not be effective in treatment of cancer. Presence of EGFRvVI may indicate that the subject will not be responsive to therapeutic regimens comprising molecules that target the ligand binding domain. Accordingly, detection of the presence of EGFRvVI may direct those skilled in the art to rule out treatment regimens that comprise EGFR inhibitors.

### EXAMPLES

### Example 1: Identification of novel EGFR variant

In this example, a glioma cerebrospinal fluid (CSF) sample was obtained from a patient (Patient ID UCS-0001). The biopsy was EGFRvIII positive. Microvesicles were extracted from 4 ml of CSF sample. Nucleic acids, for example RNA, were extracted by methods known in the art.

Two EGFR PCR-based detection assays were performed. In the EGFRvIII-specific assay, the following primers and probe were used:
EGFR Forw 1 - CTGCTGGCTGCGCTCTG (SEQ ID NO: 6)
EGFRv3 rev4 - CGTGATCTGTCACCACATAATTACC (SEQ ID NO: 7)
EGFR probe 6 - TTCCTCCAGAGCCCGACT (FAM-dye labeled Minor Grove Binder (MGB) probe) (SEQ ID NO: 8)

In the second EGFR PCR-based detection assay, the following primers and probe were used:
EGFR Forw 1 - CTGCTGGCTGCGCTCTG (SEQ ID NO: 6)
EGFR Rev 1 - TTCCTCCATCTCATAGCTGTCG (SEQ ID NO: 9)
EGFR probe 6 - TTCCTCCAGAGCCCGACT (FAM-dye labeled Minor Grove Binder (MGB) probe) (SEQ ID NO: 8)

The EGFRvIII specific assay (EGFR Forw 1, EGFRv3rev4, EGFR probe 6) did not produce an amplification product from the CSF sample. However, the second EGFR assay (EGFR Forw 1, EGFR Rev 1 and EGFR probe 6) produced an amplification product.

This amplification product from the second EGFR assay was further investigated by a qualitative PCR amplification. The following PCR setup was used:

| | |
|---|---|
| EGFR Forw 1 (18uM) | 1ul |
| EGFR Rev 1 (18uM) | 1ul |
| dNTPs (10mM) | 1ul |
| HF enzyme buffer (5×) | 10ul |
| Phusion hot start II DNA polymerase | 0.5ul |
| DMSO | 1.5ul |
| H2O | 30ul |
| cDNA | 5ul |

The PCR cycling conditions were as follows:
1. 98° C, 30 sec
2. 98° C, 10 sec
3. 62° C, 15 sec
4. 72° C, 15 sec
5. 72° C, 5 min
Repeat step 2-4 for 36 cycles.

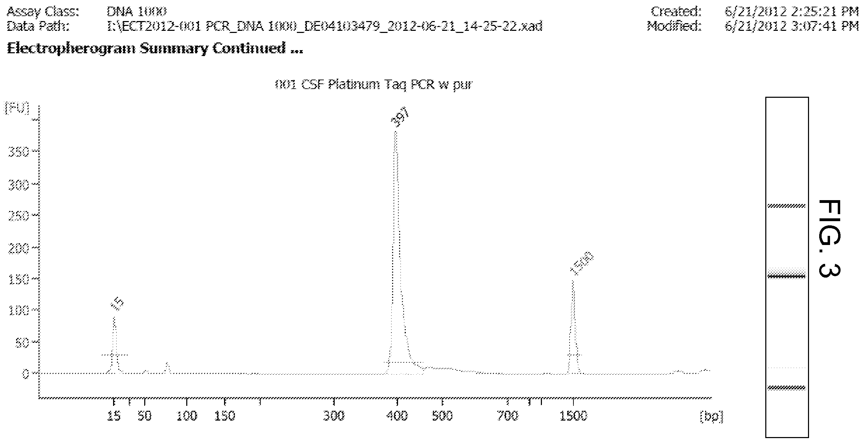

The amplified PCR product from the EGFR assay showed a larger band than EGFRvIII when analyzed. Bioanalyzer results were generated into an electropherogram, as shown in Figure 3. The amplified PCR product was cloned into a TOPO vector and sequenced. The nucleotide sequence of exons 1, 2, part of 3, part of 7 and 8 is shown in Figure 4.

Sequencing of the amplified PCR product identified a break a third into exon 3 that joins halfway into exon 7. EGFRvIII is a deletion in which the whole exon 2-7 is deleted, wherein the DNA break can be anywhere in the intronic regions. In contrast, the newly identified EGFR variant is characterized by a DNA break that occurs within the exons, and thus, has a defined junction on DNA. EGFRvVI contains a deletion that is 10730 bp long, and includes coding and non-coding regions. EGFRvVI nucleotide sequence encodes a polypeptide that lacks amino acids 90-221 of the WT EGFR, as shown in Figure 5.

Additional examples of primers that can be used for amplification of EGFRvVI in addition to EGFR Forw1 (SEQ ID NO: 6) and EGFR Rev1 (SEQ ID NO: 9) are as follows:
EGFR qPCR exon 3-7 del forw 1: TGGTCCTTGGGAATTTGGAA (SEQ ID NO: 10) EGFR
qPCR exon 3-7 del rev: GTGGGGTTGTAGAGCATGAGGA (SEQ ID NO: 11) EGFR
qPCR exon 3-7 del probe (FAM-MGB): TACCTATGTGCAGAGGAA (SEQ ID NO: 12)

## Claims

1. An isolated epithelial growth factor receptor (EGFR) protein comprising an in-frame deletion that starts in exon 3 and ends in exon 7 of wild-type EGFR, wherein amino acids 90-221 of wild-type EGFR have been deleted and wherein said EGFR protein comprises the amino acid sequence of SEQ ID NO: 5 or an amino acid sequence having greater than 90% identity to the amino acid sequence of SEQ ID NO: 5.

2. An isolated polynucleotide coding for the EGFR protein of claim 1.

3. An isolated polynucleotide coding for epithelial growth factor receptor (EGFR) protein, wherein said polynucleotide comprises the nucleotide sequence of SEQ ID NO: 3.

4. An isolated polynucleotide coding for epithelial growth factor receptor (EGFR) protein, wherein said polynucleotide comprises the nucleotide sequence of SEQ ID NO: 4.

5. An antibody that specifically binds to an EGFR protein encoded by a polynucleotide comprising the nucleotide sequence of SEQ ID NO: 3 or SEQ ID NO: 4, or that binds an EGFR protein comprising the amino acid sequence of SEQ ID NO: 5 or an amino acid sequence having greater than 90% identity to the amino acid sequence of SEQ ID NO: 5, but does not bind to wild-type EGFR and other EGFR variants.

6. An *in vitro* method for diagnosing cancer in a subject comprising analyzing a biological sample for the presence of the EGFR protein or polynucleotide of any one of claims 1-4, wherein presence of the EGFR protein or polynucleotide indicates the presence of a cancer or a higher predisposition of the subject to develop a cancer.

7. The method of claim 6, wherein the cancer is a neurologic cancer or an epithelial cancer, optionally wherein the neurologic cancer is glioblastoma or glioblastoma multiforme, or wherein the epithelial cancer is colon cancer, lung cancer, prostate cancer, breast cancer or other solid tumor cancers.

8. An *in vitro* method for assessing the responsiveness of a subject to a therapeutic regimen comprising analyzing a biological sample from a subject and determining the presence, absence, or level of expression of the EGFR protein or polynucleotide of any one of claims 1-4, wherein the presence, absence, or level of expression of said EGFR protein or polynucleotide indicates the responsiveness of the subject to the therapeutic regimen, optionally wherein the therapeutic regimen comprises an EGFR inhibitor.

9. An *in vitro* method for recommending a therapeutic regimen comprising analyzing a biological sample from a subject, determining the presence of the EGFR protein or polynucleotide of any one of claims 1-4, wherein the presence of said EGFR protein or polynucleotide rules out a therapeutic regimen comprising an EGFR inhibitor that binds to or targets amino acids 90-221 of wild-type EGFR protein.

10. The method of any of claims 8 or 9, wherein said EGFR inhibitor is a RNAi agent or antibody.

11. An agent that specifically binds to an EGFR protein encoded by a polynucleotide comprising the nucleotide sequence of SEQ ID NO: 3 or SEQ ID NO: 4, or that binds an EGFR protein comprising the amino acid sequence of SEQ ID NO: 5 or an amino acid sequence having greater than 90% identity to the amino acid sequence of SEQ ID NO: 5, but does not bind the wild-type EGFR and other EGFR variants, for use in treating cancer, wherein said agent is an antibody.

12. The agent for the use of claim 11, wherein said antibody is covalently linked to a therapeutic agent.

13. The agent for the use of claim 12, wherein said therapeutic agent is a chemotherapeutic agent, an anti-cancer agent, a toxin, a radioisotope, or a nanoparticle.

14. A specific inhibitor of an EGFR protein encoded by a polynucleotide comprising the nucleotide sequence of SEQ ID NO: 3 or SEQ ID NO: 4, or that inhibits an EGFR protein comprising the amino acid sequence of SEQ ID NO: 5 or an amino acid sequence having greater than 90% identity to the amino acid sequence of SEQ ID NO: 5, but does not inhibit wild-type EGFR and other EGFR variants for use in treating a cancer wherein said inhibitor is a RNAi agent or antibody.

## Patentansprüche

1. Isoliertes epitheliales Wachstumsfaktorrezeptor (EGFR)-Protein, umfassend eine In-frame-Deletion, die in Exon 3 beginnt und in Exon 7 vom Wildtyp-EGFR endet, wobei Aminosäuren 90-221 vom Wildtyp-EGFR deletiert wurden und wobei das EGFR-Protein die Aminosäuresequenz SEQ ID NR: 5 oder eine Aminosäuresequenz mit einer Identität von mehr als 90% gegenüber der Aminosäuresequenz SEQ ID NR: 5 umfasst.

2. Isoliertes Polynucleotid, das für das EGFR-Protein nach Anspruch 1 codiert.

3. Isoliertes Polynucleotid, das für epitheliales Wachstumsfaktorrezeptor (EGFR)-Protein codiert, wobei das Polynucleotid die Nucleotidsequenz SEQ ID NR: 3 umfasst.

4. Isoliertes Polynucleotid, das für epitheliales Wachstumsfaktorrezeptor (EGFR)-Protein codiert, wobei das Polynucleotid die Nucleotidsequenz SEQ ID NR: 4 umfasst.

5. Antikörper, der spezifisch an ein EGFR-Protein bindet, das durch ein Polynucleotid codiert ist, umfassend die Nucleotidsequenz SEQ ID NR: 3 oder SEQ ID NR: 4, oder der an ein EGFR-Protein bindet, umfassend die Aminosäuresequenz SEQ ID NR: 5 oder eine Aminosäuresequenz mit einer Identität von mehr als 90% gegenüber der Aminosäuresequenz SEQ ID NR: 5, der aber nicht an Wildtyp-EGFR und andere EGFR-Varianten bindet.

6. In-vitro-Verfahren zur Diagnose von Krebs bei einem Subjekt, umfassend die Analyse einer biologischen Probe auf das Vorhandensein des EGFR-Proteins oder -Polynucleotids nach einem der Ansprüche 1-4, wobei das Vorhandensein des EGFR-Proteins oder -Polynucleotids das Vorhandensein von Krebs oder einer höheren Prädisposition des Subjekts für die Entwicklung von Krebs anzeigt.

7. Verfahren nach Anspruch 6, wobei der Krebs ein neurologischer Krebs oder ein Epithelkrebs ist, wobei wahlweise der neurologische Krebs ein Glioblastom oder Glioblastoma multiforme ist oder wobei der Epithelkrebs Dickdarmkrebs, Lungenkrebs, Prostatakrebs, Brustkrebs oder ein anderer solider Tumorkrebs ist.

8. In-vitro-Verfahren zur Beurteilung der Ansprechempfindlichkeit eines Subjekts auf ein therapeutisches Regime, umfassend die Analyse einer biologischen Probe von einem Subjekt, und Bestimmung des Vorhandenseins, Fehlens oder Expressionsniveaus des EGFR-Proteins oder -Polynucleotids nach einem der Ansprüche 1-4, wobei das Vorhandensein, Fehlen oder Expressionsniveau des EGFR-Proteins oder -Polynucleotids die Ansprechempfindlichkeit des Subjekts auf das therapeutische Regime anzeigt, wobei wahlweise das therapeutische Regime einen EGFR-Inhibitor umfasst.

9. In-vitro-Verfahren zum Empfehlen eines therapeutischen Regimes, umfassend die Analyse einer biologischen Probe von einem Subjekt, Bestimmung des Vorhandenseins des EGFR-Proteins oder -Polynucleotids nach einem der Ansprüche 1-4, wobei das Vorhandensein des EGFR-Proteins oder - Polynucleotids ein therapeutisches Regime, umfassend einen EGFR-Inhibitor, der an Aminosäuren 90-221 vom Wildtyp-EGFR-Protein bindet oder auf diese abzielt, ausschließt.

10. Verfahren nach einem der Ansprüche 8 oder 9, wobei der EGFR-Inhibitor ein RNAi-Wirkstoff oder -Antikörper ist.

11. Wirkstoff, der spezifisch an ein EGFR-Protein bindet, das von einem Polynucleotid codiert ist, umfassend die Nucleotidsequenz SEQ ID NR: 3 oder SEQ ID NR: 4, oder der an ein EGFR-Protein bindet, umfassend die Aminosäuresequenz SEQ ID NR: 5 oder eine Aminosäuresequenz mit einer Identität von mehr als 90% gegenüber der Aminosäuresequenz SEQ ID NR: 5, der aber nicht an den Wildtyp-EGFR und andere EGFR-Varianten bindet, zur Verwendung bei der Krebsbehandlung, wobei der Wirkstoff ein Antikörper ist.

12. Wirkstoff zur Verwendung nach Anspruch 11, wobei der Antikörper kovalent mit einem therapeutischen Wirkstoff verbunden ist.

13. Wirkstoff zur Verwendung nach Anspruch 12, wobei der therapeutische Wirkstoff ein chemotherapeutischer Wirkstoff, ein Antikrebswirkstoff, ein Toxin, ein Radioisotop oder ein Nanopartikel ist.

14. Spezifischer Inhibitor eines EGFR-Proteins, das von einem Polynucleotid codiert ist, umfassend die Nucleotidsequenz SEQ ID NR: 3 oder SEQ ID NR: 4, oder der ein EGFR-Protein hemmt, umfassend die Aminosäuresequenz SEQ ID NR: 5 oder eine Aminosäuresequenz mit einer Identität von mehr als 90% gegenüber der Aminosäuresequenz SEQ ID NR: 5, der aber nicht Wildtyp-EGFR und andere EGFR-Varianten hemmt, zur Verwendung bei der Krebsbehandlung, wobei der Inhibitor ein RNAi-Wirkstoff oder -Antikörper ist.

## Revendications

1. Protéine isolée récepteur du facteur de croissance épidermique (EGFR) comprenant une délétion en phase qui débute dans l'exon 3 et finit dans l'exon 7 de l'EGFR de type sauvage, où les acides aminés 90-221 de l'EGFR de type sauvage ont été délétés et où ladite protéine EGFR comprend la séquence d'acides aminés SEQ ID N° : 5 ou une séquence d'acides aminés identique à plus de 90 % à la séquence d'acides aminés SEQ ID N° : 5.

2. Polynucléotide isolé codant pour la protéine EGFR de la revendication 1.

3. Polynucléotide isolé codant pour une protéine récepteur du facteur de croissance épidermique (EGFR), où ledit polynucléotide comprend la séquence nucléotidique SEQ ID N° : 3.

4. Polynucléotide isolé codant pour une protéine récepteur du facteur de croissance épidermique (EGFR), où ledit polynucléotide comprend la séquence nucléotidique SEQ ID N° : 4.

5. Anticorps qui se lie spécifiquement à une protéine EGFR codée par un polynucléotide comprenant la séquence nucléotidique SEQ ID N° : 3 ou SEQ ID N° : 4 ou qui se lie à une protéine EGFR comprenant la séquence d'acides aminés SEQ ID N° : 5 ou une séquence d'acides aminés identique à plus de 90 % à la séquence d'acides aminés SEQ ID N° : 5, mais qui ne se lie pas à l'EGFR de type sauvage ou à d'autres variants de l'EGFR.

6. Procédé *in vitro* pour le diagnostic d'un cancer chez un sujet, comprenant l'analyse d'un échantillon biologique pour identifier la présence de la protéine EGFR ou du polynucléotide de l'une quelconque des revendications 1-4, où la présence de la protéine EGFR ou du polynucléotide indique la présence d'un cancer ou une plus forte prédisposition du sujet à développer un cancer.

7. Procédé de la revendication 6, où le cancer est un cancer neurologique ou un cancer épithélial, éventuellement où le cancer neurologique est un glioblastome ou un glioblastome multiforme, ou où le cancer épithélial est un cancer du côlon, un cancer du poumon, un cancer de la prostate, un cancer du sein ou d'autres cancers à tumeurs solides.

8. Procédé *in vitro* pour l'évaluation de l'aptitude d'un sujet à répondre à un schéma thérapeutique, comprenant l'analyse d'un échantillon biologique obtenu d'un sujet et la détermination de la présence, de l'absence ou du niveau d'expression de la protéine EGFR ou du polynucléotide de l'une quelconque des revendications 1-4, où la présence, l'absence ou le niveau d'expression de ladite protéine EGFR ou dudit polynucléotide indique l'aptitude du sujet à répondre au schéma thérapeutique, éventuellement où le schéma thérapeutique comprend un inhibiteur de l'EGFR.

9. Procédé *in vitro* pour la recommandation d'un schéma thérapeutique, comprenant l'analyse d'un échantillon biologique obtenu d'un sujet et la détermination de la présence de la protéine EGFR ou du polynucléotide de l'une quelconque des revendications 1-4, où la présence de ladite protéine EGFR ou dudit polynucléotide élimine un schéma thérapeutique comprenant un inhibiteur de l'EGFR qui se lie aux acides aminés 90-221 de la protéine EGFR de type sauvage ou qui vise lesdits acides aminés.

10. Procédé de l'une quelconque des revendications 8 ou 9, où ledit inhibiteur de l'EGFR est un agent ARNi ou un anticorps.

11. Agent qui se lie spécifiquement à une protéine EGFR codée par un polynucléotide comprenant la séquence nucléotidique SEQ ID N° : 3 ou SEQ ID N° : 4 ou qui se lie à une protéine EGFR comprenant la séquence d'acides aminés SEQ ID N° : 5 ou une séquence d'acides aminés identique à plus de 90 % à la séquence d'acides aminés SEQ ID N° : 5 mais qui ne se lie pas à l'EGFR de type sauvage ou à d'autres variants de l'EGFR pour une utilisation dans le traitement d'un cancer, où ledit agent est un anticorps.

12. Agent pour l'utilisation de la revendication 11, où ledit anticorps est lié de manière covalente à un agent thérapeutique.

13. Agent pour l'utilisation de la revendication 12, où ledit agent thérapeutique est un agent chimiothérapeutique, un agent anticancéreux, une toxine, un radio-isotope ou une nanoparticule.

14. Inhibiteur spécifique d'une protéine EGFR codée par un polynucléotide comprenant la séquence nucléotidique SEQ ID N° : 3 ou SEQ ID N° : 4 ou qui inhibe une protéine EGFR comprenant la séquence d'acides aminés SEQ ID N° : 5 ou une séquence d'acides aminés identique à plus de 90 % à la séquence d'acides aminés SEQ ID N° : 5 mais qui n'inhibe pas l'EGFR de type sauvage et d'autres variants de l'EGFR pour une utilisation dans le traitement d'un cancer, où ledit inhibiteur est un agent ARNi ou un anticorps.
